# EUROPEAN PATENT APPLICATION

(11) **EP 0 617 979 A1**
(43) Date of publication of application: **05.10.1994**
(21) Application number: 94500057.8
(22) Date of filing: 28.03.1994
(51) Int. Cl.: A61N 1/30, A61N 1/20, A61N 1/26

(54) **Manual device for carrying out an iontophoresis treatment**

(30) Priority: 02.04.1993 ES 9300918
(71) Applicant: Villar Faro, Juan Jose, E-08036 Barcelona (ES)
(72) Inventor: Villar Faro, Juan Jose, E-08036 Barcelona (ES)
(74) Representative: Sanchez del Campo Gonzalez de Ubierna, Ramon

(57) **Abstract**

A manual device for treating iontophoresis consisting of a source of low voltage direct current housed in a manual operated case (5) containing cells (1) and (2), in which the grasping portion (6) for the user's hand (10) communicates with one of the terminals of the source of direct current, while the othet terminal contacts an insulated applicator head (3), so an iontophoresis circuit being made through the user's hand and body until the contacting portion with the applicator head, the direct current source being connected, via one of its terminals, to the applicator head, and via the other terminal to the electric conductive manual grasping portion.

## Description

### BACKGROUND OF THE INVENTION

This specification refers to a manual device for treating iontophoresis by means of which the iontophoresis treatment can be manually carried out by the own user.

### FIELD OF THE INVENTION

The present invention applies to the industry devoted to the manufacture of ancillary apparatuses for Medicine, and it can be punctually used in the cosmetic field.

### RELATED ART

Iontophoresis is a treatment starting from the application of an electric current to a zone on the human body, from which it is desired to attain a specially efficient absorption of medical and/ or cosmetic substances.

The iontophoresis application envisages to apply a low voltage direct current on a given human body surface, so avoiding every kind of possible aggressive actions on the user's skin, the application of the electric current having a determined polarity, that is to say, positive or negative, being combined with the own polarity of the medical or cosmetic products to be applied, which is to be inverse to that of the electric current applied.

In this way, the permeation absorption normally produced between the'substance deposited on the skin and the blood vessels located under the epidermis is enhanced by the repulsion action between the electric current passing through the desired corporal zone and the own polar particles contained in the product to be diffussed.

Iontophoresis is performed, up till now, by means of apparatuses having some magnitude and under clinical or aesthetic treatments in specialized laboratories or surgeries.

The apparatuses utilized are fed by the mains electric current that is to say, alternating current and, after the voltage of this alternating current is rectified and regulated, it is applied by an adequate means to the body area on which the treatment is to be performed.

Nevertheless, the use of these special apparatuses of some magnitude limits the possibility of applying an iontophoresis treatment and increases its cost.

An evident solution to this problem would be to rely on a device allowing the iontophoresis treatment to be applied by the own user, that is to say, a manual operated device used by the own - user, so making possible to appreciably extend the use of said treatment.

### SUMMARY OF THE INVENTION

The manual device for treating iontophoresis as proposed by the invention is defined as a device allowing a user to carry out, starting from it, a treatment performed by himself directly without the need of going to any medical center where these treatments are usually carried out, with a consequent substantial saving in - costs, which, at present, are relatively high with regard to said treatments.

In a most definite manner, the manual device for treating iontophoresis, which is the object of the invention, is configured starting from a source of direct current which can be manually operated; for example, a small case containing low voltage electric cells or batteries, one of their terminals being coupled to an applicator which makes contact with the user's skin at the desired zone, the other contact being made by a conductive zone to the user's hand, which will allow the electric circuit to make through the user's body between the contact portion of the applicator head and the hand of the own user grasping the iontophoresis device.

Of course, the physical operation of the device will vary within ample limits, the above mentioned conditions being met.

When using a source of low voltage direct current, which is easily attained by-using 1,5V cells or batteries, the iontophoresis treatment can be accomplished without the need of more or less complex mechanisms for transforming and rectifying the alternating - current of the mains, as it is usual at present.

Also, the user will avail himself of simple and economic means, of easy transportation and selfcontained with regard to the electric current feed, so that said treatment may be applied in any moment and place.

### BRIEF DESCRIPTION OF THE DRAWINGS

In order to complement this description and to aid to a better understanding of the features of the invention, the accompanying - drawings, which are a part of this specification, show in an illustrative but non limitative manner, the following:

Figure 1 shows a sectional view of a manual device for treating iontophoresis according to the invention.

Figure 2 shows a schematic view of the application of the device shown in Figure 1.

Figure 3 shows, lastly, a schematic view of the electric circuit arrangement during the treatment.

### DESCRIPTION OF A PREFERRED EMBODIMENT

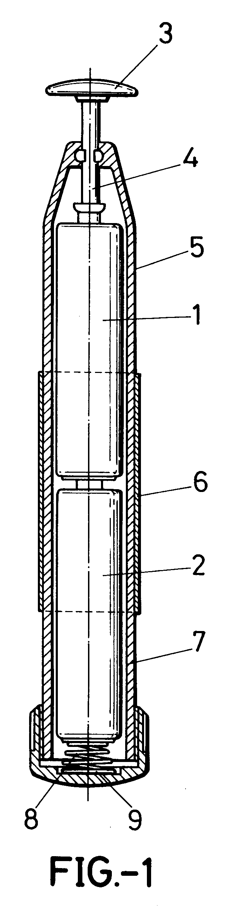

From Figure 1, it can be seen the manner in which the manual device for treating iontophoresis envisaged is constituted starting from a source of direct current of a desired voltage, which, in the case illustrated in said Figure, includes electric cells 1, 2, in a variable number, to attain a low voltage of direct - application, said cells being coupled in series and a terminal of same, for example, the positive terminal, being connected to an applicator 3 having a plate o lightly bulged cap shape in contact with the cells by variable means, for example, through a rod 4.

The cells are contained within a manual case of easy grasping 5, having insulating characteristics, with an outside portion 6 of conductive type in contact, through variable means shown in the Figure by a bridge 7, with the base of cell 2 by means of a spring 8 and a contact plate 9, so that the user, when grasping manually the device, at least at the conductive portion 6, makes the circuit through his body, so carrying out the iontophoresis treatment.

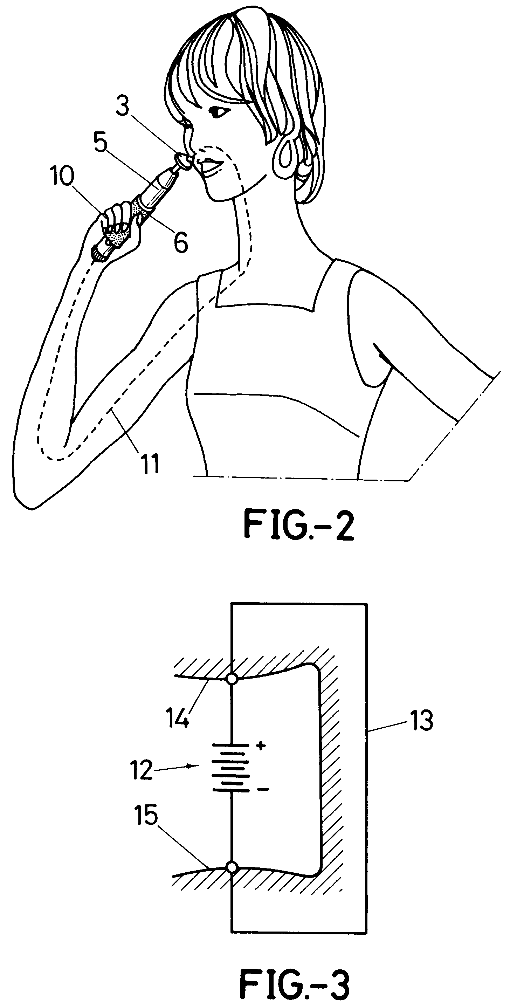

Such as is seen in Figure 2, the user's hand 10 grasps the iontophoresis device so that the applicator 3 makes contact with the user body zone where the treatment is to be applied.

The electric circuit will make through the user's body after attaining the iontophoresis effect on the corporal zone desired, the path of the electric current being illustrated by means of a dotted line 11:

Therefore, in a simplified way, such as is shown in Figure 3, a direct current generator 12 will make an electric current according to a circuit, schematically represented under reference 13, between two surfaces 14 and 15 of the user's body, one of which will be his own hand, and the other surface will be the application zone on his body.

It is not considered necessary to extend more this description for an expert in the art to understand the scope of the invention and the advantages derived from it.

The materials, shape, size and arrangement of the elements are always open to variation, provided that it does not imply any alteration to the essence of the invention.

The terms under which this specification has been described - should be always taken in an ample and non limitative sense.

## Claims

**1.** A manual device for treating iontophoresis, characterized in that it is constituted starting from a source of low voltage direct current (1) and (2), housed in a manual operated case (5) in which the grasping portion (6) for an user's hand (10) communicates with one of the terminals of the source of direct current, while the other terminal contacts an applicator head (3), which is insulated with regard to said grasping portion (6), so allowing an iontophoresis circuit to be made through the user's hand (10) and the user's body until the portion contacting the applicator head (3).

**2.-** A manual device for treating iontophoresis, according to - claim 1, characterized in that the source of direct current formed by cells or accumulators (1) and (2) is connected via one of their terminals to the applicator head (3), while via the other terminal is connected to the manual grasping portion, which is conductive.

**3.-** A manual device for treating iontophoresis, according to any of the previous claims, characterized in that the cells or - accumulators (1) and (2) are contained in a case (5) having isolating features, the grasping portion of same being made of an electricity conductive material, and being connected to one of the terminals of the assembly of cells or accumulators, while the other terminal is connected to the applicator which has a lightly bulged tip structure (3).
